# EUROPEAN PATENT APPLICATION

(11) **EP 2 433 597 A1**
(43) Date of publication of application: **28.03.2012**
(21) Application number: 09844903.6
(22) Date of filing: 20.05.2009
(51) Int. Cl.: A61F 13/15, A61F 13/49, A61F 13/494, A61F 13/56, A61F 13/66

(54) **DISPOSABLE DIAPER AND DIAPER OUTER COVER**

(71) Applicant: Koyo Corporation, Kanagawa 247-0014 (JP)
(72) Inventor: TAKAGI Takamasa, Yokohama-shi Kanagawa 236-0004 (JP); MITSUGI Takehisa, Yokohama-shi Kanagawa 236-0004 (JP); SHIMEZAKI Yukio, Yokohama-shi Kanagawa 236-0004 (JP)
(74) Representative: Schaeberle, Steffen
(86) International application number: PCT/JP2009/059251
(87) International publication number: WO 2010/134169

(57) **Abstract**

The present invention provides a disposable diaper and a diaper cover having inner seal parts which can prevent body wastes from leaking out of both sides of the diaper or both sides of the diaper cover. It is provided with a liquid permeable topsheet 2, a liquid impermeable backsheet 3, an absorber 4 which absorbs and holds liquid and is interposed between the topsheet and the backsheet, a belt 7 to which one end of the topsheet and backsheet is fixed, a connection 8 which is formed at one end of the belt and is connected with the other end of the belt, inner seal parts 5 and 6 provided at both sides of the topsheet in the lengthwise direction and arranged to be stretchable in the lengthwise direction, and fastening portions 5c and 6c fastened to the belt and provided at front ends of the inner seal parts.

## Description

### Technical Field

The present invention relates to a disposable diaper and a diaper cover, more particularly to a disposable diaper and a diaper cover for preventing body wastes from leaking out of both sides. (In this specification the term "both sides" includes "both sides" literally and "either side".)

### Background Art

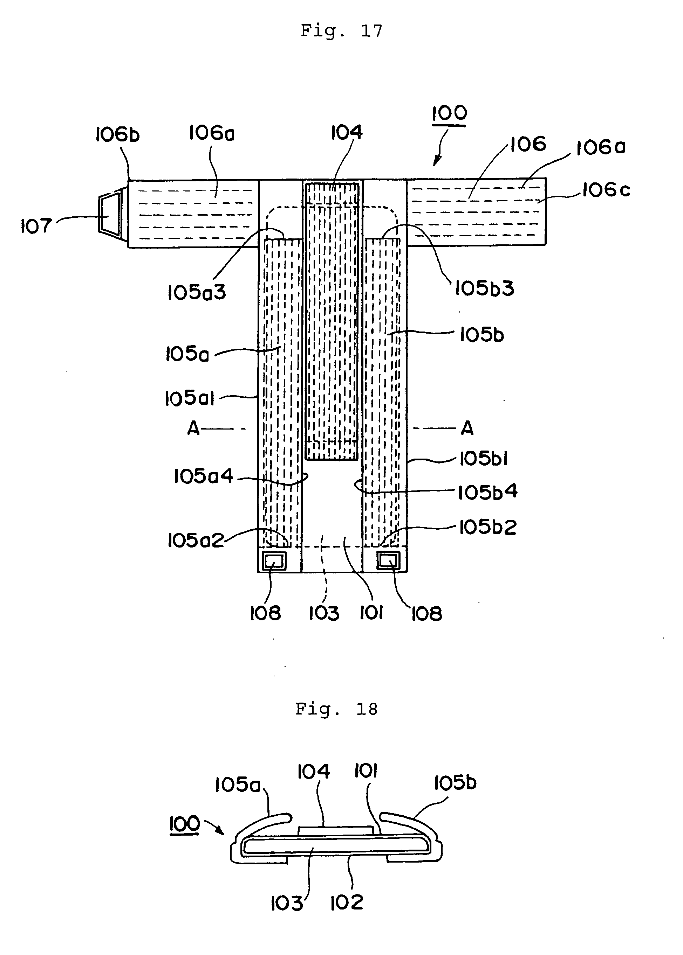

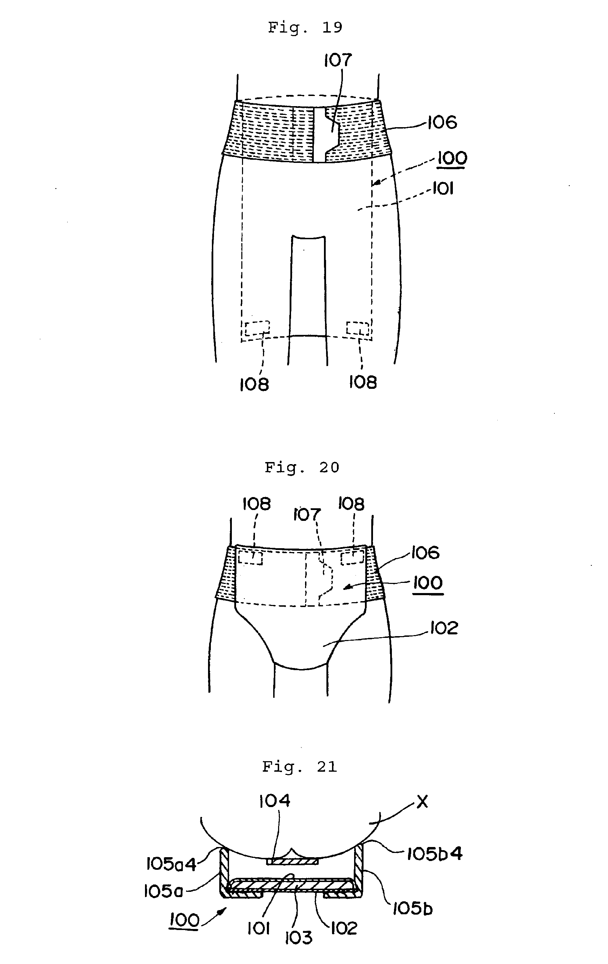

In a conventional disposable diaper and a conventional diaper cover, there has been a need for preventing body wastes from leaking. To cope with the need, various improvements have been made. With reference to FIGS. 17 to 21, a disposable diaper shown in Patent Document 1 will be described by way of example. It should be noted that FIG. 17 is a plan view of the disposable diaper shown in Patent Document 1, FIG. 18 is a section along the line A-A shown in FIG. 17, FIGS. 19 and 20 are views for explaining a method of putting on the disposable diaper, and FIG. 21 is a sectional view showing a situation where the disposable diaper is in use.

A disposable diaper 100 is provided with a liquid permeable topsheet 101, a liquid impermeable backsheet 102, and an absorber 103 which absorbs and holds liquid and is interposed between the above-mentioned topsheet 101 and backsheet 102. Further, an upper surface sheet 104 is provided on an upper surface of the above-mentioned topsheet 101.
Furthermore, the above-mentioned disposable diaper 100 has barrier cuffs 105a and 105b at both sides of the topsheet 101 in the lengthwise direction. These barrier cuffs 105a and 105b are for preventing the body wastes from leaking out of diaper side edges. In particular, groups of barrier cuffs 105a and 105b respectively have fixed-ends 105a1, 105a2, 105a3, 105bl, 105b2, and 105b3 and free ends 105a4 and 105b4 which are joined to both sides, a front end, and a rear end of the topsheet 101.

Further, this diaper 100 has a belt 106 connected to a rear end side of the backsheet 102. This belt 106 is extended in a lateral direction of the backsheet 102 from the rear end side of the backsheet 102. It should be noted that, an elastic member 106a stretchable along an extending direction is provided in the belt 106.
Furthermore, a connection 107, such as a hook and loop fastener, is formed at one end 106b of the belt 106, and this connection 107 allows the one end 106b of the above-mentioned belt 106 to be attachable to and detachable from the other end 106c.
Still further, in this diaper 100, fastening portions 108 are provided on a surface near front ends of the barrier cuffs 105a and 105b.

A method of using this diaper 100 will be described with reference to FIGS. 19 and 20.
First, as shown in FIG. 19, the diaper 100 is arranged to a wearer's back side, and the belt 106 is wound around a wearer's waist. Then, the belt 106 is positioned by connecting the connection 107 to the other end 106c.
Subsequently, the front end side of the diaper 100 is pulled up to a wearer's abdomen side through the crotch and the fastening portion 108 is fastened to the belt 106, so that the wearer puts on the diaper 100.

In such a diaper 100, as shown in FIG. 21, since the barrier cuffs 105a and 105b are provided at both sides of the topsheet 101 in the lengthwise direction, the above-mentioned barrier cuffs 105a and 105b rise, and their tips 105a4 and 105b4 come into contact with buttocks X when the above-mentioned diaper 100 is worn.
As a result, the above-mentioned barrier cuffs 105a and 105b prevent the body wastes from leaking out of both sides of the diaper.

### Prior Art

### Patent Document

Patent Document 1: Japanese Patent Application Publication No. 2008-43525

### SUMMARY OF THE INVENTION

### Problems to Be Solved by the Invention

As described above, the conventional disposable diaper is such that the whole diaper 100 having barrier cuffs 105a and 105b at both ends of the above-mentioned topsheet 101 is pressed against the body to close a gap and prevent leakage.
Incidentally, as described above, the front end side of the diaper 100 (topsheet 101) is fixed to the belt 106 by fastening the above-mentioned fastening portion 108 to the belt 106.
On the other hand, since the front and rear ends (fixed ends 105a2 , 105a3, 105b2, 105b3) of the above-mentioned barrier cuffs are fixed to the topsheet 101 which is considerably influenced by movement of the diaper, there is a problem that the movement of the diaper (position shift of the topsheet 101) causes the barrier cuffs 105a and 105b to move, thus generating a gap between the body and the above-mentioned barrier cuffs.
For example, when the heavy body wastes are discharged into the diaper 100, the topsheet 101 and the backsheet 102 fall down due to the weight, and the barrier cuffs 105a and 105b whose front and rear ends are fixed to the topsheet 101 fall together with the backsheet 102.
As a result, a gap is generated between the buttocks X and the barrier cuffs 105a and 105b, and there is a problem that the body wastes cannot be prevented from leaking out of both sides of the diaper.

Further, in order to prevent the body wastes from leaking out of both sides of the diaper, it is possible that the front end side of the diaper may be pulled up strongly to the abdomen side through the crotch when putting on the diaper 100, and the fastening portion 108 provided for the topsheet 101 may be fastened to the belt 106.
However, since the fastening portion 108 fastened to the belt 106 is formed on the surface of the topsheet 101, the above-mentioned barrier cuffs 105a and 105b whose front and rear ends are fixed to the topsheet 101 are inclined inwardly if the front end side of the diaper is pulled up strongly and the fastening portion 108 is fastened to the belt 106. Thus, there is a problem that the body wastes cannot be prevented from leaking out of both sides of the diaper. These problems arise with not only the diaper but also the diaper cover.

In order to solve these problems, the present inventors have diligently studied to find that inner seal parts which are stretchable in the lengthwise direction are formed at both sides of the above-mentioned diaper or the diaper cover and the above-mentioned inner seal parts are brought into close contact with the buttocks, so that the body wastes may be prevented from leaking out of both sides of the diaper and both sides of the diaper cover.
The present invention has been made in order to solve the above-mentioned technical problems, and aims at providing the disposable diaper and the diaper cover having the inner seal parts which can prevent the body wastes from leaking out of both sides of the diaper or the diaper cover further.

### Means for Solving the Problems

The present invention has been made in order to achieve the above-mentioned aim and the diaper in accordance with the present invention is characterized by having a liquid permeable topsheet, a liquid impermeable backsheet, an absorber which absorbs and holds liquid and is interposed between the above-mentioned topsheet and backsheet, a belt to which one end of the above-mentioned topsheet and backsheet is fixed, a connection which is formed at one end of the above-mentioned belt and is connected with the other end of the belt, stretchable inner seal parts which are provided at both sides of the above-mentioned topsheet or backsheet and elongated in the lengthwise direction, and a fastening portion fastened to the above-mentioned belt provided on an upper surface of a front end of the above-mentioned inner seal part.

In this diaper, pulling the inner seal parts (bringing the inner seal parts into close contact with the buttocks), the front end side of the diaper is passed through the crotch towards the wearer' s abdomen side to fasten the above-mentioned fastening portion to the belt, thus putting on the diaper.
Therefore, in such a diaper, when the above-mentioned diaper is worn, the inner seal parts come into close contact with the buttocks and the body wastes can be prevented from leaking out of both sides of the diaper further.
In addition, the belt is fixed to a hipbone side and therefore prevented from slipping downwardly, and the inner seal parts which are stretched and in close contact with the buttocks are fastened to the belt, whereby a gap is unlikely to arise between the buttocks and the inner seal parts, even if shift arises in the diaper (topsheet, absorber), and it is possible to prevent the body wastes from leaking out of both sides of the diaper.

Here, it is desirable that the fastening portions are provided at the front end of the above-mentioned topsheet separately from the fastening portions of the above-mentioned inner seal parts.
Further, it is desirable that the above-mentioned inner seal part has a rising portion which is raised from the upper surface of the above-mentioned topsheet and a contacting part which extends outwardly from a tip of the above-mentioned rising portion, and the fastening portion of the above-mentioned inner seal part is provided on an upper surface at a front end of the above-mentioned contacting part.

Furthermore, it is preferable that a height of the front end in which the fastening portion is provided is equal to or greater than a height of the rear end on a belt side in the rising portion of the above-mentioned inner seal part.
The rear ends of the inner seal parts maybe firmly pressed against the body, or weight is applied when on wearer's back, they may fall on an upper surface of the absorber. Therefore, in the case where the inner seal parts at the rear ends are high, the inner seal parts may cover the upper surface of the absorber, and an absorption area may be decreased to lead to absorption inhibition, which may cause the leakage.
Thus, it is desirable that the height of rising portions of the rear ends of the above-mentioned inner seal parts is reduced.
On the other hand, since the front ends of the inner seal parts are zones where urine is discharged and a space is needed where the inner seal part serves as a breakwater for preventing the flowing urine from leaking. Thus, it is desirable that the inner seal parts at the front ends are high. Further, the urine-absorbed diaper is heavy and therefore tends to act to separate the whole diaper from the body.
In order to ease such a stress, the height of the inner seal parts is increased so as to prevent a gap which may cause the leakage. Thus, it is desirable that the height of the rising portions of the front end of the above-mentioned inner seal parts is large.
Therefore, as for the height of the rising portions of the above-mentioned inner seal parts, it is desirable that the height at the front ends in which the fastening portions are provided is greater than that at the rear ends on the belt side.

Further, it is desirable that a cut-out is formed between the rising portion at the front end of the above-mentioned inner seal part and an upper surface of the topsheet.
The diaper is used by various types of people, from one who is bedridden to one who can move. When the diaper is used by one who can move, the diaper is subjected to various types of stresses. The inner seal part is also influenced by motion of the main part of the diaper, and therefore may tend to produce a gap which leads to the leakage.
As described above, the influence of the various stresses concerning the inner seal can be eased by providing the cut-out at the front end of the inner seal. Naturally, however, a position where the cut-out is formed must avoid an area into which urine flows.

The present invention arises in order to achieve the above-mentioned aim, and the disposable diaper in accordance with the present invention is characterized by having a liquid permeable topsheet, a liquid impermeable backsheet, an absorber which absorbs and holds liquid and is interposed between the above-mentioned topsheet and backsheet, a belt to which one end of the above-mentioned topsheet and backsheet is fixed, a connection which is formed at one end of the above-mentioned belt and is connected with the other end of the belt, stretchable parts attached to both sides of the above-mentioned topsheet and elongated in the lengthwise direction, stretchable inner seal parts attached to sides of the above-mentioned stretchable parts and elongated in the lengthwise direction, and a fastening portion fastened to the above-mentioned belt provided on the topsheet side of a front end of the above-mentioned inner seal part.

In this diaper, the belt is fixed to the hipbone side and therefore prevented from slipping downwardly, and the inner seal parts which are stretched and in close contact with the buttocks are fastened to the belt, whereby a gap is unlikely to arise between the buttocks and the inner seal parts, even if shift arises in the diaper (topsheet, absorber), and it is possible to prevent the body wastes from leaking out of both sides of the diaper.
Further, since the fastening portions are provided on the topsheet side at tips of the above-mentioned inner seal parts, even if the front end side of the diaper is pulled up strongly to fasten the fastening portions to the belt, the stretchable parts and the inner seal parts do not incline inwardly but rise certainly, thus preventing the body wastes from leaking out of both sides of the diaper.

Here, it is desirable that in order to avoid the gap the above-mentioned inner seal part is made of a hydrophobic or water-repellent melt-blown nonwoven fabric whose material is elastic or a liquid impermeable elastomer of polystyrene, polyurethane, polyester, polyolefin, etc.
Further, the above-mentioned inner seal part may be obtained by bonding a stretchable nonwoven fabric or a stretchable elastomer (of polystyrene, polyurethane, polyester, polyolefin, etc.) to a hydrophobic or water-repellent inelastic nonwoven fabric so as to be stretchable. Furthermore, it may be obtained by corrugating the hydrophobic or water-repellent inelastic nonwoven fabric so as to be stretchable.
By "corrugating" is meant a process of making a nonwoven fabric uneven to have a shape of bellows and a stretching function.

The present invention arises in order to achieve the above-mentioned aim, and the diaper cover in accordance with the present invention is characterized by having a liquid impermeable sheet, a belt to which one end of the above-mentioned sheet is fixed, a connection which is formed at one end of the above-mentioned belt and is connected with the other end of the belt, inner seal parts elongated in the lengthwise direction on both sides of the above-mentioned sheet, and a fastening portion fastened to the above-mentioned belt provided on a topsheet side of a front end of the above-mentioned inner seal part.

In this diaper cover, pulling the inner seal parts (bringing the inner seal parts into close contact with the buttocks), the front end side of the diaper cover is passed through the crotch towards the wearer's abdomen side to fasten the above-mentioned fastening portion to the belt, thus putting on the diaper cover.
Therefore, in such a diaper cover, when the above-mentioned diaper is worn, the inner seal parts come into close contact with the buttocks and the body wastes can be prevented from leaking out of both sides of the diaper further.
In addition, the belt is fixed to the hipbone side and therefore prevented from slipping downwardly, and the inner seal parts which are stretched and in close contact with the buttocks are fastened to the belt, whereby a gap is unlikely to arise between the buttocks and the inner seal parts, even if shift arises in the diaper, and it is possible to prevent the body wastes from leaking out of both sides of the diaper.

The present invention arises in order to achieve the above-mentioned aim, and the diaper cover in accordance with the present invention is characterized by having a liquid impermeable sheet, a belt to which one end of the above-mentioned sheet is fixed, a connection which is formed at one end of the above-mentioned belt and is connected with the other end of the belt, stretchable parts elongated in the lengthwise direction at both sides of the above-mentioned sheet, longitudinally stretchable inner seal parts elongated in the lengthwise direction at the sides of the above-mentioned stretchable parts, and a fastening portion fastened to the above-mentioned belt provided on a sheet side of a front end of the above-mentioned inner seal part.

In this diaper cover, the belt is fixed to the hipbone side and therefore prevented from slipping downwardly, and the inner seal parts which are stretched and in close contact with the buttocks are fastened to the belt, whereby a gap is unlikely to arise between the buttocks and the inner seal parts, even if shift arises in the diaper, and it is possible to prevent the body wastes from leaking out of both sides of the diaper.
Further, since the fastening portions are provided on the sheet side at the tips of the above-mentioned inner seal parts, even if the front end side of the diaper is pulled up strongly to fasten the fastening portions to the belt, the stretchable parts and the inner seal parts do not incline inwardly but rise certainly, thus preventing the body wastes from leaking out of both sides of the diaper cover.

Here, it is desirable that in order to avoid the gap the above-mentioned inner seal part is made of a hydrophobic or water-repellent melt-blown nonwoven fabric whose material is elastic or an elastomer of polystyrene, polyurethane, polyester, polyolefin, etc.
Further, the above-mentioned inner seal part may be obtained by bonding a stretchable member (a stretchable nonwoven fabric or an elastomer of polystyrene, polyurethane, polyester, polyolefin, etc.) to a hydrophobic or water-repellent inelastic nonwoven fabric so as to be stretchable.
Furthermore, the above-mentioned inner seal part may be obtained by corrugating the hydrophobic or water-repellent inelastic nonwoven fabric so as to be stretchable.
By "corrugating" is meant a process of making a nonwoven fabric uneven to have a shape of bellows and a stretching function.

### Effect of the Invention

According to the present invention, it is possible to obtain a disposable diaper having inner seal parts which can prevent body wastes from leaking out of both sides of the diaper or a diaper cover having the inner seal parts which can prevent body wastes from leaking out of both sides of the diaper cover further.

### BRIEF DESCRIPTION OF THE DRAWINGS

[FIG. 1] FIG. 1 is a perspective view showing a first preferred embodiment in accordance with the present invention.
[FIG. 2] FIG. 2 is a view for explaining a method of putting on a diaper shown in FIG. 1.
[FIG. 3] FIG. 3 is a sectional view showing a wearing situation of the diaper shown in FIG. 1.
[FIG. 4] FIG. 4(a) is a schematic representation for explaining a fastening state of an inner seal part, and FIG. 4 (b) is a schematic representation for explaining a fastening state of conventional barrier cuffs.
[FIG. 5] FIGS. 5 are views showing a modification of the first preferred embodiment, and showing a modification of a rising portion of the inner seal part.
[FIG. 6] FIG. 6 is a plan view showing a second preferred embodiment in accordance with the present invention.
[FIG. 7] FIG. 7 is a view for explaining a method of putting on the diaper shown in FIG. 6.
[FIG. 8] FIG. 8 is a sectional view showing a wearing situation of the diaper shown in FIG. 6.
[FIG. 9] FIG. 9 is a plan view showing a third preferred embodiment in accordance with the present invention.
[FIG. 10] FIG. 10 is a section on the plane I-I shown in FIG. 9.
[FIG. 11] FIG. 11 is a sectional view showing a wearing situation of the diaper shown in FIG. 9.
[FIG. 12] FIG. 12 is a plan view showing a fourth preferred embodiment in accordance with the present invention.
[FIG. 13] FIG. 13 is a section on the plane II-II shown in FIG. 12.
[FIG. 14] FIG. 14 is a sectional view showing a wearing situation of the diaper shown in FIG. 12.
[FIG. 15] FIG. 15 is a plan view showing a fifth preferred embodiment in accordance with the present invention.
[FIG. 16] FIG. 16 is a view showing a modification of the first to fifth preferred embodiments in accordance with the present invention.
[FIG. 17] FIG. 17 is a plan view of a disposable diaper shown in Patent Document 1.
[FIG. 18] FIG. 18 is a section along the line A-A shown in FIG. 17.
[FIG. 19] FIG. 19 is a view for explaining a method of putting on the disposable diaper shown in FIG. 17.
[FIG. 20] FIG. 20 is a view for explaining a method of putting on the disposable diaper shown in FIG. 17.
[FIG. 21] FIG. 21 is a sectional view showing a situation where the disposable diaper shown in FIG. 17 is in use.

### Explanation of References Signs

10, 20, 30: disposable diapers
12, 21, 31: topsheets
13, 22, 32: backsheets
4, 23, 33: absorbers
5, 11, 25, 34, 42: inner seal parts
6, 12, 26, 35, 43: inner seal parts
5a, 6a: rising portions
5b, 6b: contacting parts
5c, 6c: fastening portions
7: belt
8: connection
9: fastening portion
13: fastening portion
24: stretchable part
25: stretchable part
40: diaper cover
41: sheet
46: fastening portion

### BEST MODE FOR CARRYING OUT THE INVENTION

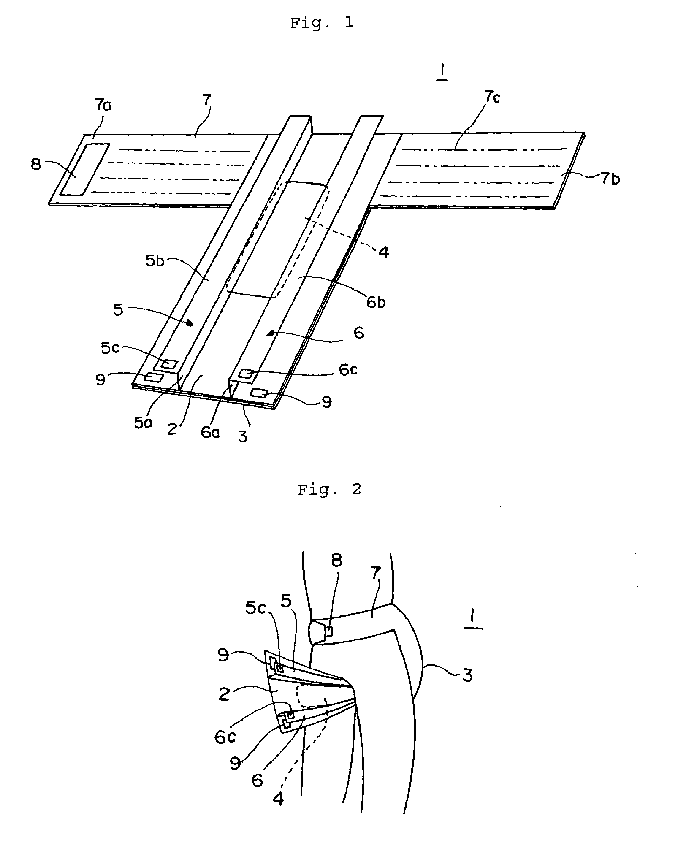

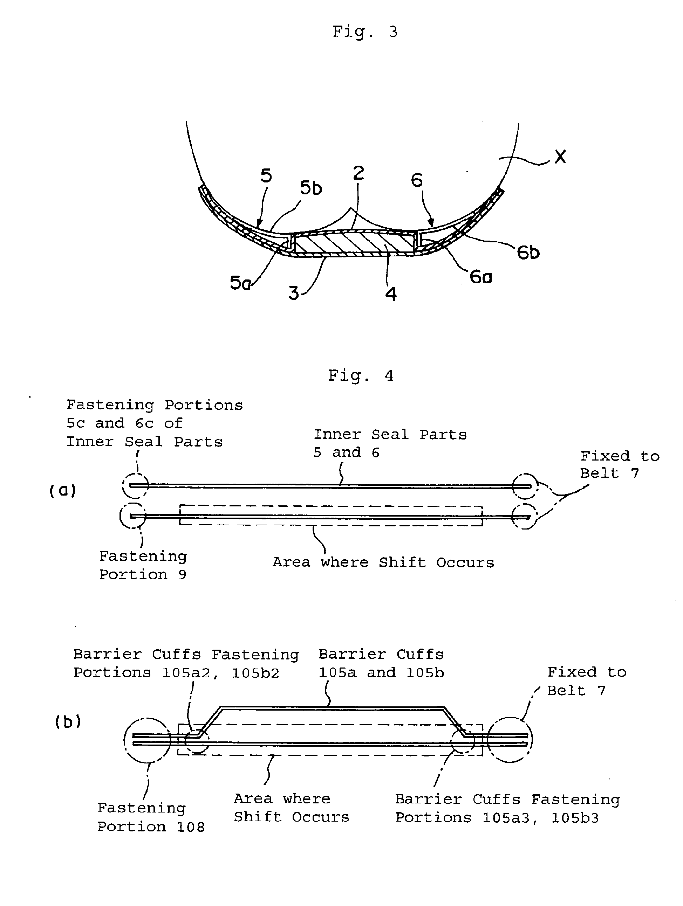

A first preferred embodiment of a disposable diaper in accordance with the present invention will be described with reference to FIGS. 1 to 4. It should be noted that FIG. 1 is a perspective view showing the first preferred embodiment in accordance with the present invention, FIG. 2 is a view for explaining a method of putting on a diaper shown in FIG. 1, FIG. 3 is a sectional view showing a wearing situation of the diaper shown in FIG. 1, and FIG. 4 is a schematic representation for explaining a fastening state of an inner seal part.
As shown in FIG. 1, a disposable diaper 1 is provided with a liquid permeable topsheet 2, a liquid impermeable backsheet 3, and an absorber 4 which absorbs and holds liquid and is interposed between the above-mentioned topsheet 2 and the backsheet 3.

This topsheet 2 is for passing liquid body waste therethrough into the absorber 4 and holding solid body waste.
For such a topsheet 2, it is possible to use a hydrophilic nonwoven fabric or textile, and a porous plastic film, etc., which have been used conventionally, as well as a hydrophobic nonwoven fabric having openings, a film having openings, etc.

Further, the backsheet 3 is for preventing the body wastes absorbed in the absorber 4 from passing and leaking.
For such a backsheet 3, it is possible to use a hydrophobic nonwoven fabric, a liquid impermeable film, a sheet in which a hydrophilic nonwoven fabric and a liquid impermeable film are laminated together, a water-repellent nonwoven fabric, and a moisture permeable plastic film having pores, which have been used conventionally, or a laminate thereof. Among these, the moisture permeable material is preferred, since sweatiness when worn can be reduced and displeasure to a wearer can also be reduced.

Furthermore, the absorber 4 is for absorbing and holding the body wastes. For such an absorber 4, it is possible to use one having fluff pulp and a super absorbent polymer in combination which have been used conventionally and one in which a cellulose fiber, a thermoplastic resin, a super absorbent polymer, a thermal adhesive fiber, etc. are mixed together and heat-treated.

Still further, in the disposable diaper 1, inner seal parts 5 and 6 elongated in the lengthwise direction are provided on both sides of an upper surface of the topsheet 2.
These inner seal parts 5 and 6 have rising portions 5a and 6a which are raised from the upper surface of the topsheet 2 and contacting parts 5b and 6b which extend outwardly from tips of the above-mentioned rising portions 5a and 6a and are brought into close contact with buttocks X.

These inner seal parts 5 and 6 are arranged to be stretchable in the lengthwise direction. The above-mentioned inner seal parts 5 and 6 come into close contact with the buttocks X when worn, to thereby prevent the body wastes from leaking out of both sides of the diaper.
It is desirable that a material for these inner seal parts 5 and 6 is hydrophobic or water-repellent and the material itself is elastic. For example, a stretchable nonwoven fabric which is manufactured by way of a meltblowing process using an elastomer resin made of polystyrene, polyurethane, polyester, polyolefin, etc., liquid impermeable urethane foam, a urethane film, an olefin elastomer, a rubber/latex elastomer, etc. are used.

Further, the above-mentioned inner seal part may be obtained by bonding a stretchable member (a stretchable nonwoven fabric, a urethane material, etc.) to a hydrophobic or water-repellent inelastic nonwoven fabric so as to be stretchable.
Furthermore, it may be obtained by corrugating the hydrophobic or water-repellent inelastic nonwoven fabric so as to be stretchable. By "corrugating" is meant a process of making a nonwoven fabric (for example) uneven to have a shape of bellows and a stretching function.
It should be noted that use of the moisture permeable material is preferred, since sweatiness when worn can be reduced and displeasure to the wearer can also be reduced.

The rear end of the above-mentioned topsheet 2 and backsheet 3 is joined to a belt 7. The above-mentioned inner seal parts 5 and 6 are extended from a front end of the above-mentioned topsheet 2 and backsheet 3 towards an upper end of the above-mentioned belt 7.
Further, fastening portions 5c and 6c, such as hook and loop fasteners, are provided on upper surfaces at front ends of the above-mentioned inner seal parts 5 and 6. These fastening portions 5c and 6c are fastened to the belt 7.

Furthermore, fastening portions 9 and 9, such as hook and loop fasteners, are formed on the upper surface at the front end of the above-mentioned topsheet 2. These fastening portions 9 and 9 are fastened to the belt 7.
Conventionally, the fastening portion is provided only on the upper surface at the front end of the topsheet, and the fastening portion is fastened to the belt to put on the diaper. The present invention, however, is **characterized in that**, in addition to the fastening portions 9 and 9 of the topsheet, the fastening portions 5c and 6c of the inner seal parts 5 and 6 are formed separately from the fastening portions 9 and 9 of the above-mentioned topsheet.

Further, stretchable members 7c, such as rubber threads, are provided in the belt 7 to be expanded and contracted. It should be noted that the belt 7 may slip down from the waist if the whole belt is formed to be stretchable, and it is preferable that the stretchable members 7c are partly provided at the back side and a hipbone side of the belt to form it to be stretchable. (Furthermore, it is not necessary to form the stretchable members 7c, such as rubber threads, in the belt 7.)
Still further, a connection 8, such as a hook and loop fastener, is provided at one end 7a of the belt 7, and the one end 7a is detachably connected to the other end 7b through the connection 8.
The connection 8 is thus fastened and fixed to the other end 7b, so that this belt 7 is fitted to the hipbone side and prevented from slipping downwardly.

Now, the reason why the inner seal parts 5 and 6 are not easy to shift downwardly will be described with reference to FIG. 4.
As shown in FIG. 4(a), in this preferred embodiment, it is arranged that the fastening portions 9 and 9 on topsheet side and the fastening portions 5c and 6c of the inner seal parts 5 and 6 are separately provided and each of them is fastened to the belt 7.
Therefore, even if there arises a cause of position shift on the topsheet 5 and 6 sides (for example, in the case where the absorber 4 contains a lot of moisture, a person wearing the diaper turns over in his/her sleep, etc.), the fastening portions 5c and 6c of the inner seal parts 5 and 6 are not affected by the cause, since they are directly fastened to the belt 7. As a result, they are not easy to shift downwardly.

On the other hand, as shown in FIG. 4(b), in the conventional diaper, fastening portions (105a2 and 105b2) of barrier cuffs 105a and 105b are fastened to the upper surface of the topsheet, and a fastening portion 108 of this topsheet is fixed to the belt.
Therefore, in the case where a cause of position shift arises on the topsheets 5 and 6 side, the conventional barrier cuffs 105a and 105b tend to shift downwardly, a gap arises between the barrier cuffs 105a and 105b and the buttocks and the body wastes tends to leak from both sides of the diaper.

Next, a method of using this diaper 1 will be described with reference to FIGS. 2 and 3. Now, as shown in FIG. 2, the diaper 1 is arranged on the wearer's back side, and the belt 7 is wound around the wearer's waist. Then, the belt 7 is positioned by connecting the connection 8 to the other end 7b.
Subsequently, the front end side of the diaper 1 is passed through the crotch towards the wearer's abdomen side in such a way that the above-mentioned fastening portions 5c and 6c are fastened to the belt 7, pulling the inner seal parts 5 and 6 (bringing the contacting parts 5b and 6b of the inner seal parts into close contact with the buttocks X).
Then, pulling the topsheet 2, the fastening portions 9 and 9 on the topsheet 2 side are fastened to the belt 7, to thereby put on the diaper 1.

In such a diaper 1, as shown in FIG. 3, the inner seal parts 5 and 6 are provided in the lengthwise direction at both sides of the topsheet 2 (backsheet 3) so that the contacting parts 5b and 6b of the inner seal parts 5 and 6 come into close contact with the buttocks X when the above-mentioned diaper is worn. As a result, it is possible to prevent the body wastes from leaking out of both sides of the diaper further.
In addition, as described above, the fastening portions 5c and 6c of the inner seal parts 5 and 6 are directly fastened to the belt 7, so that they are not easy to shift downwardly and it is possible to prevent the body wastes from leaking out of both sides of the diaper.

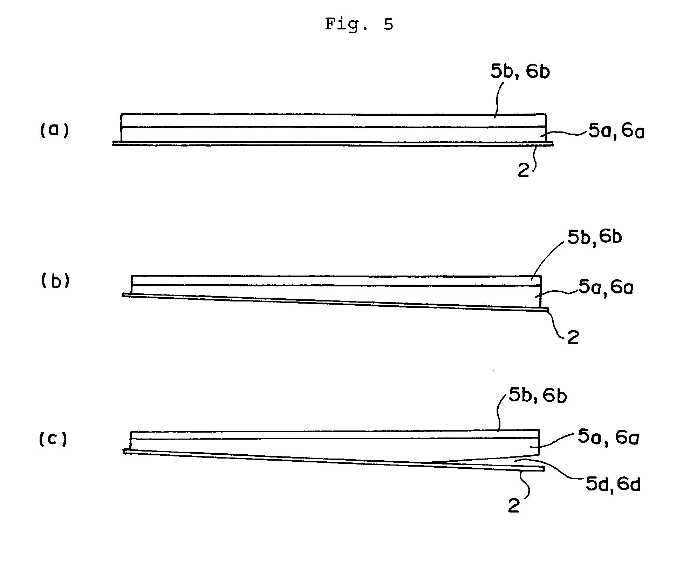

Next, a modification of this preferred embodiment is shown in FIG. 5.
As shown in FIG. 5(a), the rising portions 5a and 6a of the above-mentioned inner seal parts are formed to have a constant height from the front end to the rear end on the belt side, but it is preferable that the height on the rear end side is reduced as shown in FIG. 5(b).
The height of the rising portions 5a and 6a (at the rear end side) of the inner seal parts is thus reduced, so that a gap on the back side of the body can be reduced.
In other words, since the rear ends of these inner seal parts 5 and 6 may be firmly pressed against the body, or weight is applied when on wearer's back, they may fall on the upper surface of the absorber 4. Therefore, in the case where the rising portions of the inner seal parts 5 and 6 at the rear ends are high, the inner seal parts 5 and 6 may cover the upper surface of the absorber 4, and an absorption area may be decreased to lead to absorption inhibition, which may cause the leakage. Thus, it is desirable that the height of rising portions 5a and 6a of the rear ends of the above-mentioned inner seal parts 5 and 6 is reduced.
On the other hand, since the front ends of the inner seal parts 5 and 6 are zones where urine is discharged and a space is needed where the inner seal part serves as a breakwater for preventing the flowing urine from leaking. Therefore, it is desirable that the rising portions 5a and 6a of the inner seal parts at the front ends are high.
Further, the diaper absorbing urine is heavy and therefore tends to act to separate the whole diaper from the body. In order to ease such a stress, the height of the rising portions 5a and 6a of the inner seal parts 5 and 6 is increased so as to prevent a gap which may cause the leakage. Thus, it is desirable that the height of the rising portions 5a and 6a of the front end of the above-mentioned inner seal parts 5 and 6 is large.
Therefore, as for the height of the rising portions 5a and 6a of the above-mentioned inner seal parts 6 and 6, it is preferable that the height at the front ends in which the fastening portions 5c and 6c are provided is equal to or greater than that at the rear ends on the belt 7 side.

In particular, it is preferable that the height of rising portions 5a and 6a of the above-mentioned inner seal parts 6 and 6 is chosen within a range of between 30 mm and 70 mm (inclusive). Further, it is preferable that a width of the contacting parts 5b and 6b provided extending outwardly from the tips of the above-mentioned rising portions 5a and 6a is between 5 mm and 40 mm (inclusive).

Furthermore, as shown in FIG. 5(c), it is preferable that cut-outs 5d and 6d are provided at the front ends of the rising portions 5a and 6a of the above-mentioned inner seal parts.
Thus, the cut-outs 5d and 6d allow the fastening portions 5c and 6c to be fastened to the belt 7, without constraints of the topsheet 2 (backsheet 3). In other words, the fastening portions 5c and 6c can easily be fastened to the belt 7.
In particular, the diaper is used by various types of people, from one who is bedridden to one who can move. When the diaper is used by one who can move, the diaper is subjected to various types of stresses. The inner seal part is also influenced by motion of the main part of the diaper, and therefore may tend to produce a gap which leads to the leakage.
As described above, the influence of the various stresses concerning the inner seal can be eased by providing the cut-out at the front end of the inner seal. Naturally, however, a position where the cut-out is formed must avoid an area into which urine flows.

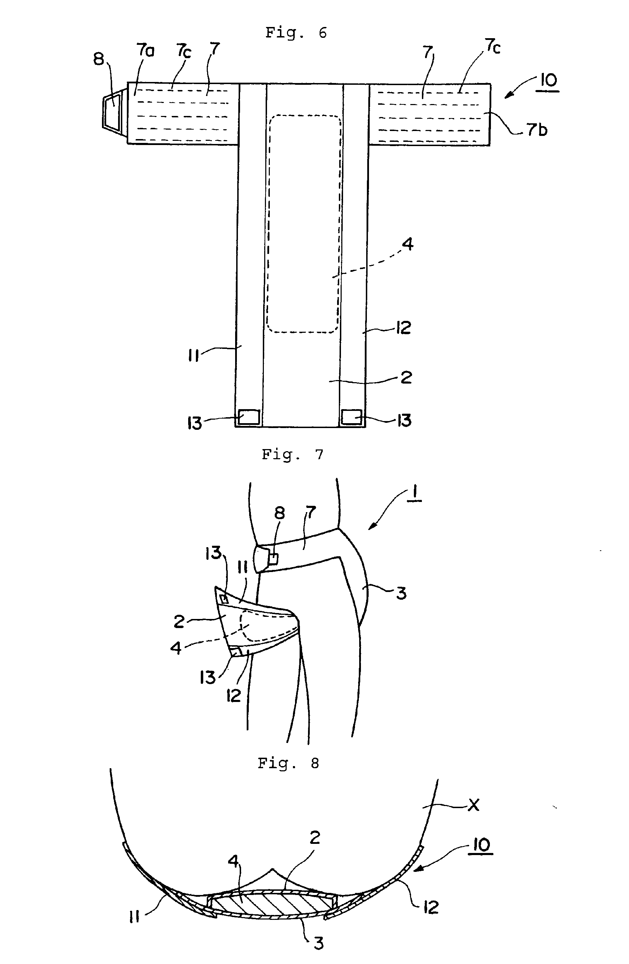

A second preferred embodiment of the disposable diaper in accordance with the present invention will be described with reference to FIGS. 6 to 8. It should be noted that FIG. 6 is a plan view showing the first preferred embodiment in accordance with the present invention, FIG. 7 is a view for explaining a method of putting on the diaper shown in FIG. 6, and FIG. 8 is a sectional view showing a wearing situation of the diaper shown in FIG. 6. In this preferred embodiment, components which are the same as or similar to those in the above-mentioned first preferred embodiment are identified using identical reference signs, and the description thereof will not be repeated.

This disposable diaper 10 is provided with inner seal parts 11 and 12 elongated in the lengthwise direction at both sides of the topsheet 2.
These inner seal parts 11 and 12 are arranged to be stretchable in the lengthwise direction. The above-mentioned inner seal parts 11 and 12 come into close contact with the buttocks X when worn, to thereby prevent the body wastes from leaking out of both sides of the diaper.

It is desirable that a material for these inner seal parts 11 and 12 is hydrophobic or water-repellent and the material itself is elastic. For example, a stretchable nonwoven fabric which is manufactured by way of a meltblowing process using an elastomer resin made of polystyrene, polyurethane, polyester, polyolefin, etc., liquid impermeable urethane foam, a urethane film, an olefin elastomer, a rubber/latex elastomer, etc. are used.
Further, the above-mentioned inner seal part may be obtained by bonding a stretchable member (a stretchable nonwoven fabric, a urethane material, etc.) to a hydrophobic or water-repellent inelastic nonwoven fabric so as to be stretchable.
Furthermore, it may be obtained by corrugating the hydrophobic or water-repellent inelastic nonwoven fabric so as to be stretchable. By "corrugating" is meant a process of making a nonwoven fabric uneven to have a shape of bellows and a stretching function.
It should be noted that use of the moisture permeable material is preferred, since sweatiness when worn can be reduced and displeasure to the wearer can also be reduced.

Sides of the above-mentioned inner seal parts 11 and 12 are joined to sides of the backsheet 3, and rear ends are joined to the belt 7. In other words, the inner seal parts 11 and 12 are joined to both sides of the backsheet 3. It should be noted that, the above-mentioned belt 7 is connected also to the rear end side of the backsheet 3.

Further, fastening portions 13 and 13, such as hook and loop fasteners, are provided on upper surfaces at front ends of the above-mentioned inner seal parts 11 and 12. These fastening portions 13 and 13 are fastened to the belt 7.
Furthermore, the connection 8, such as a hook and loop fastener, is provided at one end 7a of the belt 7, and the one end 7a is detachably connected to the other end 7b through the connection 8.
The connection 8 is thus fastened and fixed to the other end 7b, so that this belt 7 is fitted to the hipbone side and prevented from slipping downwardly. Further, the inner seal parts 11 and 12 are fastened to this belt 7 by the fastening portions 13 and 13, thus preventing a downward position shift.

A method of using this diaper 10 will be described with reference to FIGS. 7 and 8. First, as shown in FIG. 7, the diaper 1 is arranged on the wearer's back side, and the belt 7 is wound around the wearer's waist. Then the belt 7 is positioned by connecting the connection 8 to the other end 7b.
Subsequently, the front end side of the diaper 1 is passed through the crotch towards the wearer's abdomen side in such a way that the above-mentioned fastening portions 13 and 13 are fastened to the belt 7, pulling the inner seal parts 11 and 12 (bringing the inner seal parts into close contact with the buttocks), to thereby put on the diaper 1.

In such a diaper 10, as shown in FIG. 8, the inner seal parts 11 and 12 are provided in the lengthwise direction at both sides of the topsheet 2 (backsheet 3) so that the inner seal parts 11 and 12 come into close contact with the buttocks X when the above-mentioned diaper is worn. As a result, it is possible to prevent the body wastes from leaking out of both sides of the diaper further.
It should be noted that, in this preferred embodiment, one in which the sides of the inner seal parts 11 and 12 are joined to the sides of the backsheet 3 has been described, however, they may be joined to the sides of the topsheet 2.

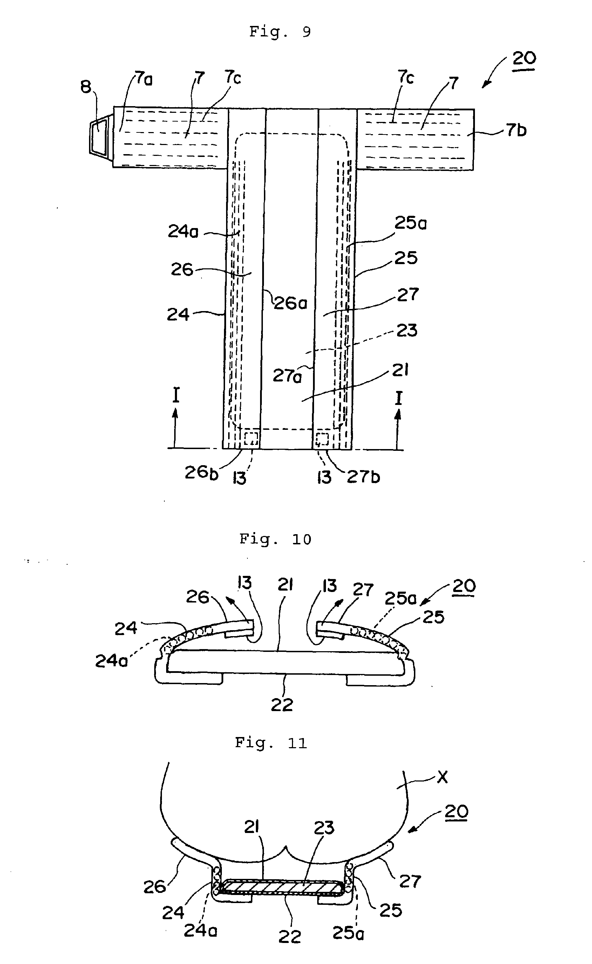

Next, a third preferred embodiment will be described with reference to FIGS. 9 to 11. It should be noted that FIG. 9 is a plan view showing the third preferred embodiment in accordance with the present invention, FIG. 10 is a section on the plane I-I shown in FIG. 9, and FIG. 11 is a sectional view showing a wearing situation of the diaper shown in FIG. 9. Further, in this preferred embodiment, components which are the same as or similar to those in the above-mentioned first and second preferred embodiments are identified using identical reference signs, and the description thereof will not be repeated.

This preferred embodiment is **characterized in that** stretchable parts 24 and 25 are formed in the lengthwise direction on both sides of a topsheet 21 of a disposable diaper 20, and inner seal parts 26 and 27 are provided in the lengthwise direction at sides of the stretchable parts 24 and 25 . Further, it is **characterized in that** the fastening portions 13 and 13, such as hook and loop fasteners, are provided on the back (topsheet 21 side) at the tip of the above-mentioned inner seal parts 26 and 27.

In other words, this disposable diaper 20 is provided with the liquid permeable topsheet 21, a liquid impermeable backsheet 22, and an absorber 23 which absorbs and holds liquid and is interposed between the above-mentioned topsheet 21 and the backsheet 22, and further provided with the stretchable parts 24 and 25 joined in the lengthwise direction on both sides of the topsheet 21.
Rubber threads 24a and 25a are accommodated in the stretchable parts 24 and 25, which are formed to be stretchable in the lengthwise direction.

The inner seal parts 26 and 27 formed of a stretchable material are joined to the stretchable parts 24 and 25 at their sides and in the lengthwise direction so as to be integral with the above-mentioned stretchable parts 24 and 25. It should be noted that tips 26a and 27a and front ends 26b and 27b of the inner seal parts 26 and 27 are free ends.
The above-mentioned stretchable parts 24 and 25 act to raise the inner seal parts 26 and 27 when worn and to bring the inner seal parts 26 and 27 into close contact with the buttocks X. The above-mentioned stretchable parts 24 and 25 and the inner seal parts 26 and 27 prevent the body wastes from leaking out of both sides of the diaper.

In particular, since the fastening portions 13 and 13 are provided on the topsheet 21 side (facing the topsheet 21) at tips of the inner seal parts 26 and 27, they act to open the above-mentioned stretchable parts 24 and 25 and the inner seal parts 26 and 27 outwardly as indicated by arrows shown in FIG. 10 when the fastening portions 13 and 13 are fastened to the belt 7.
Thus, the stretchable parts 24 and 25 and the inner seal parts 26 and 27 rise certainly, and can therefore prevent the body wastes from leaking out of both sides of the diaper.
In addition, the fastening portions 13 and 13 are provided at the stretchable inner seal parts 26 and 27 and the fastening portions 13 and 13 are fastened to the belt 7, so that a gap is unlikely to arise between the diaper and the buttocks X and it is possible to prevent the body wastes from leaking out of both sides of the diaper further.

A method of using this diaper 20 will be described.
First, as with the first preferred embodiment, the diaper 20 is arranged on the wearer's back side, and the belt 7 is wound around the wearer's waist. Then, the belt 7 is positioned by connecting the connection 8 to the other end 7b.
Subsequently, the front end side of the diaper 20 is passed through the crotch towards the wearer's abdomen side in such a way that the fastening portions 9 and 9 are fastened to the belt 7, pulling the inner seal parts 5 and 6, so that the wearer puts on the diaper 20.

In such a diaper 20, as shown in FIG. 11, the inner seal parts 26 and 27 are provided in the lengthwise direction at both sides of the topsheet 21 (backsheet 22) via the stretchable parts 24 and 25 so that the inner seal parts 26 and 27 come into close contact with the buttocks X when the above-mentioned diaper is worn. As a result, it is possible to prevent the body wastes from leaking out of both sides of the diaper further.

In particular, since the fastening portions 13 and 13 fastened to the belt 7 are provided on the topsheet 21 side at the tips of the inner seal parts 26 and 27, even if the front end side of the diaper is pulled up strongly to fasten the fastening portions 13 and 13 to the belt 7, the stretchable parts 24 and 25 and the inner seal parts 26 and 27 do not incline inwardly but rise certainly, thus preventing the body wastes from leaking out of both sides of the diaper.

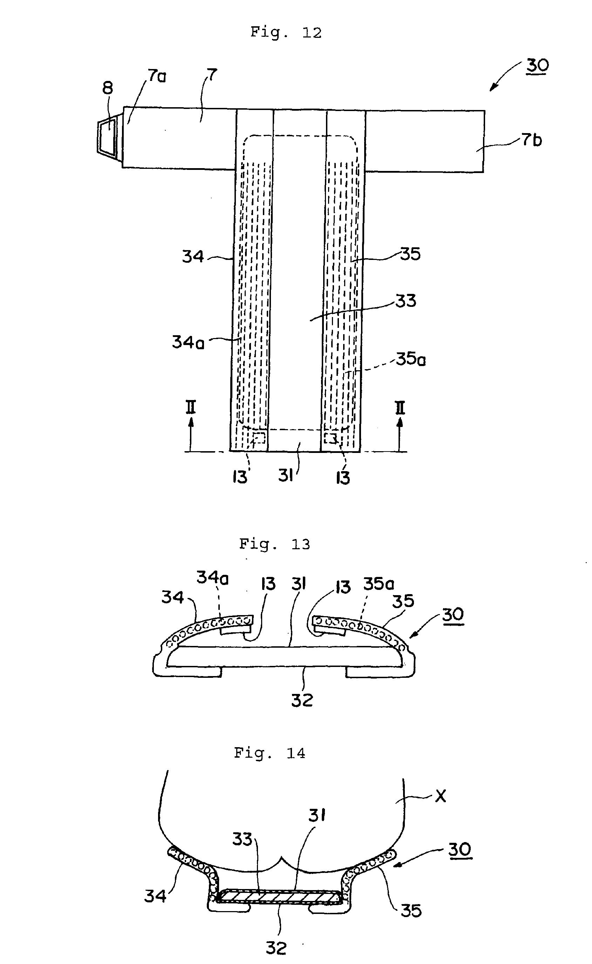

Next, a fourth preferred embodiment will be described with reference to FIGS. 12 to 14. It should be noted that FIG. 12 is a plan view showing the fourth preferred embodiment in accordance with the present invention, FIG. 13 is a section on the plane II-II shown in FIG. 12, and FIG. 14 is a sectional view showing a wearing situation of the diaper shown in FIG. 12.
This preferred embodiment is **characterized in that** inner seal parts 34 and 35 are provided in the lengthwise direction at both sides of a topsheet 31 of the disposable diaper 30, and rubber threads 34a and 35a are accommodated in the above-mentioned inner seal parts 34 and 35.

In other words, this disposable diaper 30 is provided with the liquid permeable topsheet 31, a liquid impermeable backsheet 32, and an absorber 33 which absorbs and holds liquid and is interposed between the above-mentioned topsheet 31 and the backsheet 32.
Further, the disposable diaper 30 is provided with the inner seal parts 34 and 35 elongated in the lengthwise direction at both sides of the topsheet 31.

The inner seal parts 34 and 35 are arranged to be stretchable in the lengthwise direction. The above-mentioned inner seal parts 34 and 35 come into close contact with the buttocks X when worn, to thereby prevent the body wastes from leaking out of both sides of the diaper.
The above-mentioned inner seal parts 34 and 35 are arranged to accommodate the rubber threads 34a and 35a therein so as to be stretchable.

Further, as with the above-described preferred embodiments, the connection 8, such as a hook and loop fastener, is provided at one end of the belt 7, and one end 7a is detachably connected to the other end 7b through this connection 8.
In addition, the diaper 30 further comprises the fastening portions 13 and 13 provided at the front ends of the above-mentioned inner seal parts 34 and 35. The above-mentioned fastening portions 13 and 13 are provided on the topsheet 31 side (facing the topsheet 31) at the inner seal parts 34 and 35.

A method of using this diaper 30 will be described.
First, as with the above-described preferred embodiments, the diaper 30 is arranged on the wearer's back side, and the belt 7 is wound around the wearer's waist. Then, the belt 7 is positioned by connecting the connection 8 to the other end 7b.
Subsequently, the front end side of the diaper 30 is passed through the crotch towards the wearer's abdomen side in such a way that the fastening portions 13 and 13 are fastened to the belt 7, pulling the inner seal parts 34 and 35, so that the wearer puts on the diaper 30.

In such a diaper 30, as shown in FIG. 14, the inner seal parts 34 and 35 are provided in the lengthwise direction at both sides of the topsheet 31 (backsheet 32) so that the inner seal parts 34 and 35 come into close contact with the buttocks X when the above-mentioned diaper is worn. As a result, it is possible to prevent the body wastes from leaking out of both sides of the diaper further.

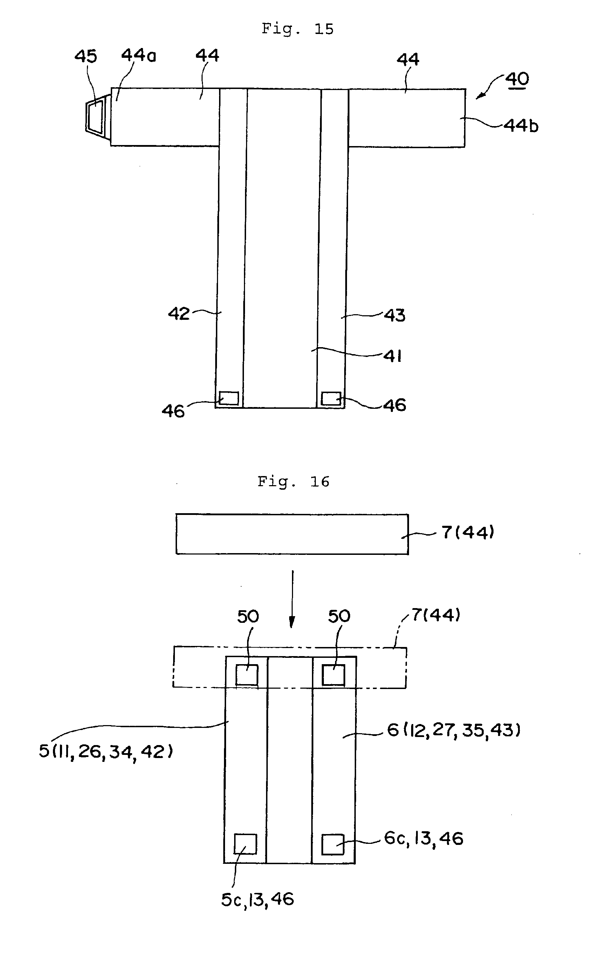

A fifth preferred embodiment in accordance with the present invention will be described with reference to FIG. 15. It should be noted that a diaper cover will be described by way of example in this preferred embodiment. FIG. 15 is a plan view showing the fifth preferred embodiment in accordance with the present invention.
As shown in FIG. 15, a diaper cover 40 is provided with a liquid impermeable sheet 41 and inner seal parts 42 and 43 elongated in the lengthwise direction at both sides of the sheet 41.

The above-mentioned sheet 41 is for covering the diaper and preventing the body wastes from passing and leaking.
For such a sheet 41, it is possible to use a liquid impermeable film, a sheet in which a hydrophilic nonwoven fabric and a liquid impermeable film are laminated together, and a moisture permeable plastic film having pores, which have been used conventionally, or a laminate thereof. Among these, the moisture permeable material is preferred, since sweatiness when worn can be reduced and displeasure to a wearer can also be reduced.

Further, these inner seal parts 42 and 43 are arranged to be stretchable in the lengthwise direction. The above-mentioned inner seal parts 42 and 43 are brought into close contact with the buttocks X when worn, to thereby prevent the body wastes from leaking out of both sides of the diaper.
It is desirable that a material for these inner seal parts 42 and 43 is hydrophobic or water-repellent and the material itself is elastic. For example, a stretchable nonwoven fabric which is manufactured by way of a meltblowing process using an elastomer resin made of polystyrene, polyurethane, polyester, polyolefin, etc., liquid impermeable urethane foam, a urethane film, an olefin elastomer, a rubber/latex elastomer, etc. are used.
Further, the above-mentioned inner seal part may be obtained by bonding a stretchable member (a stretchable nonwoven fabric, a urethane material, etc.) to a hydrophobic or water-repellent inelastic nonwoven fabric so as to be stretchable.
Furthermore, it may be obtained by corrugating the hydrophobic or water-repellent inelastic nonwoven fabric so as to be stretchable. By "corrugating" is meant a process of making a nonwoven fabric (for example) uneven to have a shape of bellows and a stretching function.

The sides of the above-mentioned inner seal parts 42 and 43 are joined to the sides of the sheet 41, and the rear ends are joined to the belt 44. In other words, the inner seal parts 42 and 43 are joined to both sides of the sheet 41. It should be noted that the above-mentioned belt 44 is also connected to the rear end side of the sheet 41.

Further, fastening portions 46 and 46, such as hook and loop fasteners, are provided on upper surfaces at front ends of the above-mentioned inner seal parts 42 and 43. The inner seal parts 42 and 43 are fastened to the belt 44 by the above-mentioned fastening portions 46 and 46, thus preventing a downward position shift.

Furthermore, a connection 45, such as a hook and loop fastener, is provided at one end of the belt 44, and one end 44a is detachably connected to the other end 44b through this connection 45. This belt 44 is fitted to the hipbone side and prevented from slipping downwardly.

A method of using of this diaper cover 40 will be described. First, putting on the diaper, the diaper cover 40 is arranged on the wearer's back side and the belt 44 is wound around the wearer's waist. Then, the belt 44 is positioned by connecting the connection 45 to the other end 44b.
Subsequently, the front end side of the diaper cover 40 is passed through the crotch towards the wearer's abdomen side in such a way that the above-mentioned fastening portions 46 and 46 are fastened to the belt 44, pulling the inner seal parts 42 and 43 (bringing the inner seal parts into close contact with the buttocks X), to thereby put on the diaper cover.

In such a diaper cover 44, the inner seal parts 42 and 43 are provided in the lengthwise direction at both sides of the sheet 41, so that the inner seal parts 42 and 43 come into close contact with the buttocks X when the above-mentioned diaper cover is worn. As a result, it is possible to prevent the body wastes from leaking out of both sides of the diaper cover further.

In addition, in the above-described fifth preferred embodiment, although the diaper cover provided with the inner seal parts in the lengthwise direction at both sides of the sheet has been described, it may be a diaper cover, like that shown in FIGS. 9 and 12, in which the stretchable parts are elongated in the lengthwise direction at both sides of the sheet and the inner seal parts (which are stretchable in the lengthwise direction) are elongated in the lengthwise direction at the sides of the above-mentioned stretchable parts.
In particular, the diaper cover is desirably provided with the liquid impermeable sheet, the belt to which one end of the above-mentioned sheet is fixed, the connection which is formed at one end of the above-mentioned belt and is connected with the other end of the belt, the stretchable parts elongated in the lengthwise direction at both sides of the above-mentioned sheet, the inner seal parts which are elongated in the lengthwise direction at the sides of the above-mentioned stretchable parts and are stretchable in the lengthwise direction, and the fastening portions fastened to the above-mentioned belt provided at the topsheet side of the front ends of the above-mentioned inner seal parts.

Further, in the above-described first through fifth preferred embodiments, the rear end sides of the inner seal parts 5, 6, 11, 12, 26, 27, 34, 35, 42, and 43 are fixed to the belts 7 and 44 side, however, fastening portions 50 like the fastening portion 5c, 6c, 13, and 46 at the front end may also be provided at the rear end side of the inner seal parts so as to be detachably fixed to the above-mentioned belts 7 and 44 as shown in FIG. 16.

## Claims

1. A disposable diaper, comprising a liquid permeable topsheet, a liquid impermeable backsheet, an absorber which absorbs and holds liquid and is interposed between said topsheet and backsheet, a belt to which one end of said topsheet and backsheet is fixed, a connection which is formed at one end of said belt and is connected with the other end of the belt, stretchable inner seal parts which are provided at both sides of said topsheet or backsheet and elongated in the lengthwise direction, and a fastening portion fastened to said belt provided on an upper surface of a front end of said inner seal part.

2. The disposable diaper as claimed in claim 1, **characterized in that** fastening portions are provided at a front end of said topsheet separately from the fastening portions of said inner seal parts.

3. The disposable diaper as claimed in any one of claims 1 to 3, **characterized in that** said inner seal part comprises a rising portion which is raised from the upper surface of said topsheet and a contacting part which extends outwardly from a tip of said rising portion, and the fastening portion of said inner seal part is provided at a front end of said contacting part.

4. The disposable diaper as claimed in claim 3, **characterized in that** a height of the front end in which the fastening portion is provided is equal to or greater than a height of the rear end on a belt side in the rising portion of said inner seal part.

5. The disposable diaper as claimed in claim 3 or 4, **characterized in that** a cut-out is formed between the rising portion at the front end of said inner seal part and an upper surface of the topsheet.

6. A disposable diaper, comprising a liquid permeable topsheet, a liquid impermeable backsheet, an absorber which absorbs and holds liquid and is interposed between said topsheet and backsheet, a belt to which one end of said topsheet and backsheet is fixed, a connection which is formed at one end of said belt and is connected with the other end of the belt, stretchable parts attached to both sides of said topsheet and elongated in the lengthwise direction, stretchable inner seal parts attached to sides of said stretchable parts and elongated in the lengthwise direction, and a fastening portion fastened to said belt provided on the topsheet side of a front end of said inner seal part.

7. The disposable diaper as claimed in claim 1 or 6, **characterized in that** said inner seal part is made of a hydrophobic or water-repellent melt-blown nonwoven fabric whose material is elastic or a liquid impermeable elastomer of polystyrene, polyurethane, polyester or polyolefin.

8. The disposable diaper as claimed in claim 1 or 6, **characterized in that** said inner seal part is obtained by bonding a stretchable member such as a stretchable nonwoven fabric or a urethane material to a hydrophobic or water-repellent inelastic nonwoven fabric so as to be stretchable.

9. The disposable diaper claimed in claim 1 or 6, **characterized in that** said inner seal part is obtained by corrugating a hydrophobic or water-repellent inelastic nonwoven fabric so as to be stretchable.

10. A diaper cover, comprising a liquid impermeable sheet, a belt to which one end of said sheet is fixed, a connection which is formed at one end of said belt and is connected with the other end of the belt, inner seal parts elongated in the lengthwise direction on both sides of said sheet, and a fastening portion fastened to said belt provided on a topsheet side of a front end of said inner seal part.

11. A diaper cover, comprising a liquid impermeable sheet, a belt to which one end of said sheet is fixed, a connection which is formed at one end of said belt and is connected with the other end of the belt, stretchable parts elongated in the lengthwise direction at both sides of said sheet, inner seal parts which are elongated in the lengthwise direction at the sides of said stretchable parts and are stretchable in the lengthwise direction, and a fastening portion fastened to said belt provided on a topsheet side of a front end of said inner seal part.

12. The diaper cover as claimed in claim 10 or 11, **characterized in that** said inner seal part is made of a hydrophobic or water-repellent melt-blown nonwoven fabric whose material is elastic or a liquid impermeable elastomer of polystyrene, polyurethane, polyester or polyolefin.

13. The diaper cover as claimed in claim 10 or 11, **characterized in that** said inner seal part is obtained by bonding a stretchable member such as a stretchable nonwoven fabric or urethane material to a hydrophobic or water-repellent inelastic nonwoven fabric so as to be stretchable.

14. The diaper cover as claimed in claim 10 or 11, **characterized in that** said inner seal part is obtained by corrugating a hydrophobic or water-repellent inelastic nonwoven fabric so as to be stretchable.
